# EUROPEAN PATENT SPECIFICATION

(11) **EP 0 655 627 B1**
(45) Date of publication and mention of the grant of the patent: **10.04.2002**
(21) Application number: 95200426.5
(22) Date of filing: 16.08.1990
(51) Int. Cl.: G01N 33/68, G01N 33/546, G01N 33/58

(54) **Kit for direct chemical binding of d-dimer from a biological sample for diagnosing and monitoring thrombolytic and hypercoagulable states**
Mittel zur direkten chemischen Bindung des D-Dimers aus einer biologischen Probe zwecks Diagnostik und Überwachung von thrombolytischen und hyperkoagulablen Zuständen
Kit pour une Méthode d'association chimique directe de dimère-D provenant d'un échantillon biologique et permettant le diagnostique et le suivi d'états de thrombolyse et d'hypercoagulabilité

(30) Priority: 17.08.1989 US 395446
(43) Date of publication of application: 31.05.1995
(62) Divisional of application: 90309018.1
(73) Proprietor: ORTHO DIAGNOSTIC SYSTEMS INC., Raritan, New Jersey 08869-0602 (US)
(72) Inventor: Thomas, Karen Ann, Somerville, New Jersey 08876 (US); Warunek, David James, East Brunswick, New Jersey 08816 (US)
(74) Representative: Mercer, Christopher Paul

(56) References cited:
- EP-A- 0 122 478
- US-A- 4 090 846
- JOURNAL OF BIOLOGICAL CHEMISTRY, vol. 254, no. 11, 10 June 1979, MD US, pages 4925-4932, XP002006463 S. A. OLEXA ET AL.: "Binding phenomena of isolated unique plasmic degradation products of human cross-linked fibrin."

## Description

This invention relates to a kit for detecting D-dimer, a fibrin breakdown product which can be indicative of disorders of the blood clotting system, which utilizes a Fragment E reagent, which directly chemically binds to D-dimer in a biological sample.

The most common disorder of the human blood clotting system is the formation of thrombi, blood clots, which when formed in vessels such as the heart can restrict blood flow and result in severe heart attacks. In addition, movement of the thrombus, or a portion of it, within the circulatory system can result in thromboembolism, by suddenly blocking blood flow. Patients receiving fibrinolytic therapy due to hypercoagulable conditions require careful monitoring of clot breakdown. Therefore, it is important to have sensitive and specific means to detect fibrinolysis, the breakdown of blood clots.

Fibrin consists of a web of branching fibers of an adhesive nature, with the power of coagulating whole blood in which it is suspended. Fibrinolysis is a system complementary to the coagulation process, and normally in equilibrium with it, disposing of deposits of fibrin. The digestion of fibrin by the enzyme plasmin produces split products including D-dimer.

In pathologic fibrinolysis, plasma concentrations of plasmin degradation products of fibrinogen and fibrin are augmented. Therefore, the presence of fibrinogen breakdown products (FDP) in human blood or urine are indicative of thrombotic disorders. FDP have been measured traditionally by agglutination assays. For example: by staphylococcal clumping; by hemagglutination immunoassay; or by the use of latex particles to which an antiserum has been attached.

Hawiger et al., "Measurement of Fibrinogen and Fibrin Degradation Products in Serum by Staphylococcal Clumping Test", J. Lab. Clin. Med. 75:93-108 (1970) disclosed a test based on the fact that a specific strain of Staphylococcus will clump in the presence of monomeric fibrin or higher molecular weight FDP. In this method, serial dilutions of serum or urine samples are mixed with suspensions of Staphylococcus cells and the presence or absence of clumping is noted.

Merskey et al., "A Rapid, Simple, Sensitive Method for Measuring Fibrinolytic Split Products in Human Serum", Proc. Soc. Exp. Biol. Med. 131:871-875 (1969) disclosed a two-step hemagglutination-inhibition test. In the first step, dilutions of the test serum sample are incubated with a dilute solution of antibodies to human fibrinogen. In the second step, unreacted antibody is detected by adding tanned red blood cells coated with human plasma. If no agglutination occurs, the sample contained sufficient FDP to neutralize the antibody.

Carney et al., "A Sensitive Latex Slide Assay for Fibrin(ogen) Fragment E", Clinical Chimica Acta 147:173-177 (1985), disclosed a latex agglutination test wherein latex particles were coated with antibodies which react directly with FDP in serum. The use of a monoclonal antibody DD-3B6/22 to D-dimer introduced the specificity required to differentiate between fibrin and fibrinogen breakdown products. This was an important improvement because detection of D-dimer, a fibrin breakdown product, is more specific for the presence of clots, which permits detection of thrombosis or hypercoagulable states with greater sensitivity and specificity than was possible with other techniques. As disclosed in an article by Elms et al., "Rapid Detection of Cross-Linked Fibrin Degradation Products in Plasma Using Monoclonal Antibody Coated Latex Particles", A.J.C.P. 85(3):360-364 (March 1986), detection of the conformational and structural changes on conversion of fibrinogen to fibrin and its cross-linking by Factor XIIIa in the process of clot formation, led to the development of new antigenic determinants that permit differentiation between their plasminolytic cleavage products. Specifically, a monoclonal antibody (DD-3B6/22) that is specific for cross-linked fibrin derivatives containing the D-dimer configuration was used in developing a latex agglutination procedure able to detect fibrin breakdown products in either plasma or serum. The sensitivity of this assay appears to be approximately 250 ng/ml of D-dimer.

Greenberg et al. in an article "Measurement of Plasma Fibrin D-dimer Levels with the Use of a Monoclonal Antibody Coupled to Latex Beads", A.J.C.P. 87(1) 94-100 (January 1987), disclosed a monoclonal antibody (DD-3B6) to fibrin D-dimer which was coupled to latex beads to provide a specific test (Dimertest) for fibrinolysis. The Dimertest assay specifically detected 2µg/ml of purified fibrin D-dimer or fibrin D-dimer/fragment E complex added to afibrinogenemic plasma.

US-A-4 090 846 discloses an indirect test for the presence of fibrinogen degradation products in human serum or urine utilizing D and E Fragments coupled to latex carrier products and an antibody specific for the D and E Fragments.

EP-A-0 122 478 discloses a method of preparing monoclonal antibody with specificity for crosslinked fibrin derivatives and an assay procedure using said antibody.

Although the agglutination assay techniques for FDP, including D-dimer, have improved, the stability and sensitivity of these techniques have not been optimized.

Accordingly, it is an object of the present invention to provide a kit for diagnosing and monitoring thrombolytic and hypercoagulable states.

The present invention relates to a kit comprising:
a reagent that specifically binds and detects the fibrin breakdown product D-dimer in a biological sample, such as plasma, urine, or other tissues or body fluids, which may contain D-dimer and other fibrin breakdown products, for diagnosing and monitoring thrombolytic and hypercoagulable states, comprising purified Fragment E bound to a solid support; and
a labelled antibody specific for Fragment D-dimer.

The present invention will become apparent from the following more detailed description.

The matrix of a blood clot or thrombus is formed by fibrin. Fibrin can be crosslinked by Factor XIIIa to form a stabilized clot. Human crosslinked fibrin is degraded by the enzyme plasmin. The D-dimer/Fragment E complex, (DD)E, is the primary soluble plasmin degradation product released from crosslinked fibrin. This complex can be further degraded by plasmin. The initial (DD)E complex contains fragments D-dimer and E₁. Upon further enzymatic digestion, Fragment E₁ is cleaved to Fragment E₂ without loss of the ability to bind to the D-dimer fragment. Digestion of Fragment E₂ to E₃ results in dissociation of the complex with the terminal digestion products of fibrin, being Fragments DD and E₃. Fragments E₁ and E₂ have the ability to bind to Fragment DD from crosslinked fibrin but do not bind with the DD-E complex, fibrinogen, or any of the plasmic degradation products of fibrinogen or of non-crosslinked fibrin.

The present invention provides a kit for detecting plasmin degradation of fibrin using purified Fragment E attached to a solid phase for a direct chemical binding of D-dimer from biological samples.

purified Fragment E can be prepared according to the methods described by Olexa and Budzynski, in Biochemistry 19, 991(1979) and in J. Biol. Chem. 254, 4925 (1979). The basic process described in these publications begins with the formation of a fibrin clot from fibrinogen enriched with Factor XIII. The clot is hydrolyzed with plasmin and the resulting digest is centrifuged to remove large clot particles. The supernatant, containing soluble degradation products including the (DD)E complex is then separated on an agarose gel bead column. The separated (DD)E complex is then incubated in a concentrated salt solution to dissociate the D-dimer and E₁ or E₂ fragments. The fragments are then separated by means of an agarose gel bead column and purified Fragment E₁ or E₂ obtained.

The kit of the invention can be used in a modified ELISA technique to quantitate the concentration of Fragment E₁/E₂, used as the capture molecule for D-dimer. In this method, Fragment E₁/E₂ is adsorbed to a solid support. A biological sample, such as plasma, urine or other body fluid containing D-dimer, is then added for binding to Fragment E₁/E₂. A monoclonal or polyclonal antibody specific for D-dimer is then conjugated to an enzyme, such as peroxidase, urease or beta-galactosidase. The conjugate (anti-D-dimer/enzyme) is then allowed to bind to the D-dimer which is directly attached to Fragment E₁/E₂. Addition of a substrate specific for the enzyme used allows for quantitation of D-dimer.

## Claims

1. A kit comprising:
a reagent that specifically binds and detects the fibrin breakdown product D-dimer in a biological sample which may contain D-dimer and other fibrin breakdown products, for diagnosing and monitoring thrombolytic and hypercoagulable states, comprising purified Fragment E bound to a solid support; and
a labelled antibody specific for Fragment D-dimer.

2. The kit of claim 1, wherein the solid support comprises latex carrier particles.

3. The kit of claim 2, wherein the latex carrier particles are amidine modified.

4. The kit of claim 2 or claim 3, further comprising a surface for mixing the reagent with the sample and for observing agglutination.

5. The kit of any one of claims 1 to 4, wherein the Fragment E is Fragment E₁.

## Patentansprüche

1. Kit, umfassend:
ein Reagenz, das spezifisch das Fibrin-Abbauprodukt D-Dimer bindet und nachweist in einer biologischen Probe, die D-Dimer und andere Fibrin-Abbauprodukte enthalten kann, zur Diagnose und der Überwachung von thrombolytischen und hyperkoagulierbaren Zuständen, umfassend gereinigtes Fragment E, das an einen festen Träger gebunden ist; und
einen für Fragment D-Dimer spezifischen markierten Antikörper.

2. Kit nach Anspruch 1, wobei der feste Träger Latex-Trägerpartikel umfaßt.

3. Kit nach Anspruch 2, wobei die Latex-Trägerpartikel Amidin-modifiziert sind.

4. Kit nach Anspruch 2 oder Anspruch 3, weiterhin umfassend eine Oberfläche zum Mischen des Reagenz mit der Probe und zur Beobachtung der Agglutination.

5. Kit nach einem der Ansprüche 1 bis 4, wobei das Fragment E Fragment E₁ ist.

## Revendications

1. Nécessaire comprenant : un réactif qui se lie spécifiquement au produit dimère D de dégradation de la fibrine et le détecte dans un échantillon biologique susceptible de contenir du dimère D et d'autres produits de dégradation de la fibrine, pour le diagnostic et le contrôle d'états thrombolytiques et hypercoagulables, comprenant du fragment E purifié fixé sur un support solide et un anticorps marqué spécifique du fragment dimère D.

2. Nécessaire selon la revendication 1, dans lequel le support solide comprend des particules de support en latex.

3. Nécessaire selon la revendication 2, dans lequel les particules de support en latex sont modifiées par de l'amidine.

4. Nécessaire selon la revendication 2 ou 3, comprenant en outre une surface pour le mélange du réactif avec l'échantillon et pour l'observation de l'agglutination.

5. Nécessaire selon l'une quelconque des revendications 1 à 4, dans laquelle le fragment E est le fragment E₁.
